# FASCICULE DE BREVET EUROPEEN

(11) **EP 0 721 989 B1**
(45) Date de publication et mention de la délivrance du brevet: **01.06.2005**
(21) Numéro de dépôt: 96400098.8
(22) Date de dépôt: 15.01.1996
(51) Int. Cl.: C12Q 1/68, C07H 21/04, C12P 19/34, C07K 14/255

(54) **Oligonucléotides pour la détection de salmonella**
Oligonukleotide für den Nachweis von Salmonella
Oligonucleotides for the detection of salmonella

(30) Priorité: 16.01.1995 FR 9500410
(43) Date de publication de la demande: 17.07.1996
(60) Demande divisionnaire: 04078201.3
(73) Titulaire: INSTITUT PASTEUR, 75724 Paris Cédex 15 (FR); INSTITUT NATIONAL DE LA SANTE ET DE LA RECHERCHE MEDICALE (INSERM), 75654 Paris Cédex 13 (FR)
(72) Inventeur: Popoff, Michel, Yvan, 78370 Plaisir (FR); Le Guern Fellous, Muriel, 92500 Rueil-Malmaison (FR)
(74) Mandataire: Desaix, Anne

(56) Documents cités:
- WO-A-92/01056
- WO-A-93/04202
- WO-A-93/18165
- WO-A-95/00664
- RES. MICROBIOLOGY, vol. 146, no. 1, Janvier 1995, pages 17-20, XP002001767 MIRAS I ET AL: "Nucleotide sequence of iagA and iagB genes involved in invasion of HeLa cells by Salmonella enterica subsp. enterica ser. Typhi"
- MOL. CELL. PROBES, vol. 7, 1993, pages 187-97, XP002001768 CHEVRIER D ET AL: "PCR product quantification by non-radioactive hybridization procedures using oligonucleotide covalently bound to microwells"
- FEMS IMMUNOLOGY AND MEDICAL MICROBIOLOGY, vol. 10, Février 1995, pages 245-52, XP002001769 CHEVRIER D ET AL: "Rapid detection of Salmonella subspecies I by PCR"
- ARRICAU ET AL: 'The RcsB-RscC regulatory system of Salmonella typhi differentially modulates the expression of invasion proteins, flagellin and Vi antigen in response to osmolarity' MOLECULAR MICROBIOLOGY vol. 29, 1998, pages 835 - 850

## Description

Le genre Salmonella contient deux espèces, Salmonella enterica, espèce divisée en six sous-espèces, sur la base de caractères biochimiques et d'homologies au niveau de l'ADN, et Salmonella bongori. Le genre est subdivisé en plus de 2000 sérovariétés définies à l'aide d'antigènes somatiques et flagellaires. Les bactéries du genre Salmonella sont de façon générale pathogènes pour l'animal ou pour l'homme. On sait ainsi que les Salmonella sont parmi les agents responsables des empoisonnements alimentaires les plus courants dans les pays développés ; c'est pourquoi des méthodes de détection rapides et fiables des sous-espèces de Salmonella sont importantes.

Les salmonelles responsables des toxi-infections alimentaires appartiennent majoritairement à la sous-espèce I (encore appelée groupe I) de S. enterica.

Les toxi-infections ne sont toutefois pas les seules pathologies provoquées par des infections par des Salmonella.

Par exemple, Salmonella enterica sous-espèce enterica sérovariété typhi (ci-dessous dénommée Typhi) est l'agent causal de la fièvre typhoïde humaine.

Compte tenu de la nature des infections provoquées par les salmonelles et de la nécessité notamment de rechercher leur présence dans les prélèvements biologiques réalisés sur des patients ou dans les aliments, il apparaît indispensable de disposer de moyens rapides et sensibles pour en détecter leur présence.

Les méthodes standard de culture largement utilisées jusqu'à présent pour la détection des salmonelles nécessitent un temps important et ne sont pas adaptées par exemple pour suivre la contamination de produits alimentaires. Pour surmonter les désavantages de ces méthodes, plusieurs méthodes fondées sur des techniques de biologie moléculaire telles que des tests d'hybridation et des tests de réaction de polymérisation en chaîne, ont d'ores et déjà été proposées. Différentes sondes d'ADN ont été utilisées dans plusieurs protocoles d'hybridation et de PCR pour détecter les sous-espèces de Salmonella dans l'alimentation. Cependant, aucune de ces techniques n'est complètement satisfaisante, puisque les séquences utilisées ne sont pas totalement connues ou pas exclusivement présentes dans le genre Salmonella et ainsi, peuvent conduire à des réactions croisées entre la sonde et des séquences d'ADN d'autres enterobactéries ou peuvent conduire à un grand nombre de faux négatifs ou de faux positifs.

Les inventeurs ont recherché des moyens permettant la détection spécifique et sensible de l'ensemble des salmonelles des espèces S. enterica et/ou S. bongori. Dans cette optique, ils se sont intéressés à la souche Salmonella enterica sous-espèce enterica sérovariété typhi (S. Typhi) et au gène participant à l'invasion de cellules par S. Typhi.

De plus, ils ont défini certaines conditions permettant la détection spécifique de groupes déterminés de Salmonelles, par exemple des bactéries du Groupe I.

On a déjà montré dans l'état antérieur de la technique, que la souche Typhi est capable d'adhérer à des monocouches de cellules HeLa et d'entrer dans ces cellules (Yabuuchi et al, 1986). Cependant, jusqu'à présent, les déterminants génétiques impliqués dans ce processus d'adhésion et d'entrée dans les cellules, n'ont pas été clairement identifiés. Elsinghorst et al (1989) ont cloné un fragment chromosomique de Typhi, qui confère à des bactéries de type Escherichia coli la capacité de pénétrer dans les cellules Henle 407. Récemment, une autre région chromosomique impliquée dans l'invasion des cellules HeLa par la souche Typhi Ty2 a été identifiée et clonée (Popoff et Dion, 1990).

Les inventeurs de la présente demande ont identifié sur un fragment d'ADN de 2,4 kb de S. typhi contenu dans la séquence HindIII de 7,9 kb décrite par Popoff et Dion (1990), des régions susceptibles de participer à l'activité d'invasion de Salmonella enterica sous-espèce enterica sérovariété Typhi dans des cellules, et en particulier dans des cultures cellulaires de type HeLa, ces régions étant susceptibles en outre d'être utilisées dans des réactions pour la réalisation d'un diagnostic généralisé de tous les représentants des espèces S. enterica et/ou S. bongori ou éventuellement dans des conditions de détection particulières, pour le diagnostic spécifique du groupe I de S. enterica.

Une séquence appelée IagA et une séquence appelée IagB ont été identifiées par les inventeurs et caractérisées comme participant à l'invasion cellulaire se manifestant lors d'une infection dûe à Salmonella enterica sous-espèce enterica sérovariété Typhi.

La spécificité de ces séquences au sein de S. Typhi a conduit les inventeurs à proposer leur utilisation pour définir des moyens pour le diagnostic d'une infection par S. typhi, voire pour le diagnostic d'une infection par des Salmonella des espèce S. enterica et/ou S. bongori ou dans certains cas pour la mise en évidence de S. enterica de groupes spécifiques.

Ces moyens utilisables pour le diagnostic d'une infection par Salmonella enterica et/ou Salmonella bongori comprennent des oligonucléotides susceptibles d'être mis en oeuvre dans des réactions d'amplification de séquences de nucléotides, par exemple des réactions de polymérisation en chaîne. L'invention se rapporte aussi à des sondes pour la détection d'acides nucléiques de S. enterica ou d'une sous-espèce spécifique de S. enterica et/ou de S. bongori, ces acides nucléiques étant le cas échéant des fragments amplifiés.

L'invention a également pour objet un kit et une méthode de détection de la présence de Salmonella enterica et/ou de Salmonella bongori dans des échantillons biologiques et par exemple, dans des produits alimentaires ou dans tout échantillon faisant l'objet d'un diagnostic clinique. Ces méthodes et kits de détection sont selon un mode de réalisation particulier de l'invention, spécifiques des souches du groupe I de S. enterica.

Selon un autre mode de réalisation de l'invention, ces méthodes permettent au contraire de rechercher la présence de bactéries S. enterica ou S. bongori du genre Salmonella. Le genre Salmonella comporte ainsi six sous-espèces ou groupes I, II, III, IV, V ou VI. Les sous-espèces I, II, III, IV et VI appartiennent à l'espèce S. enterica et la sous-espèce V appartient à l'espèce S. bongori.

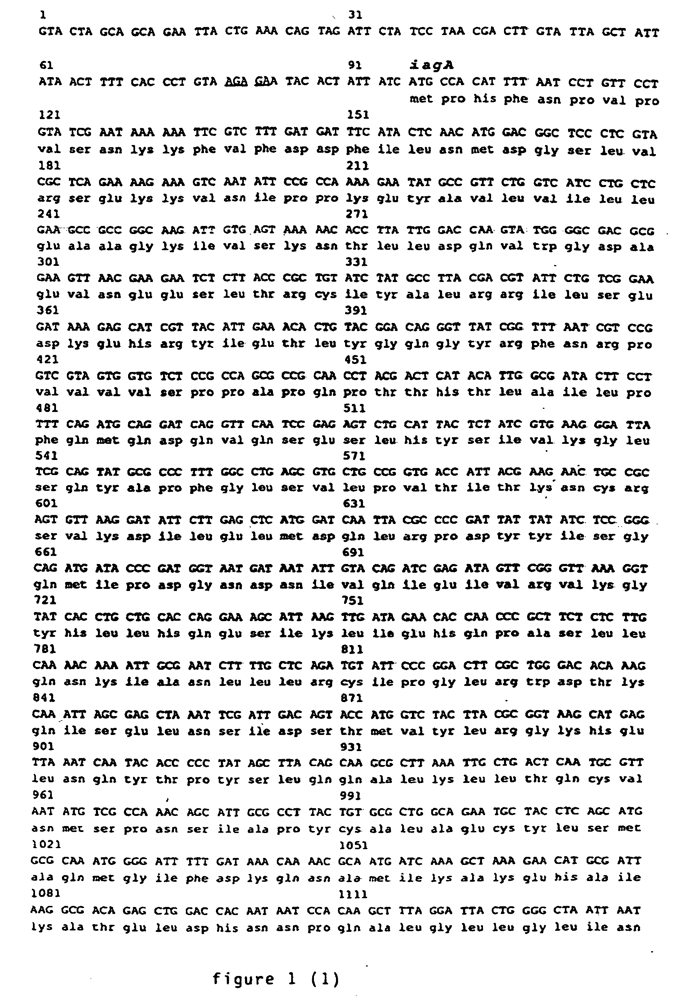

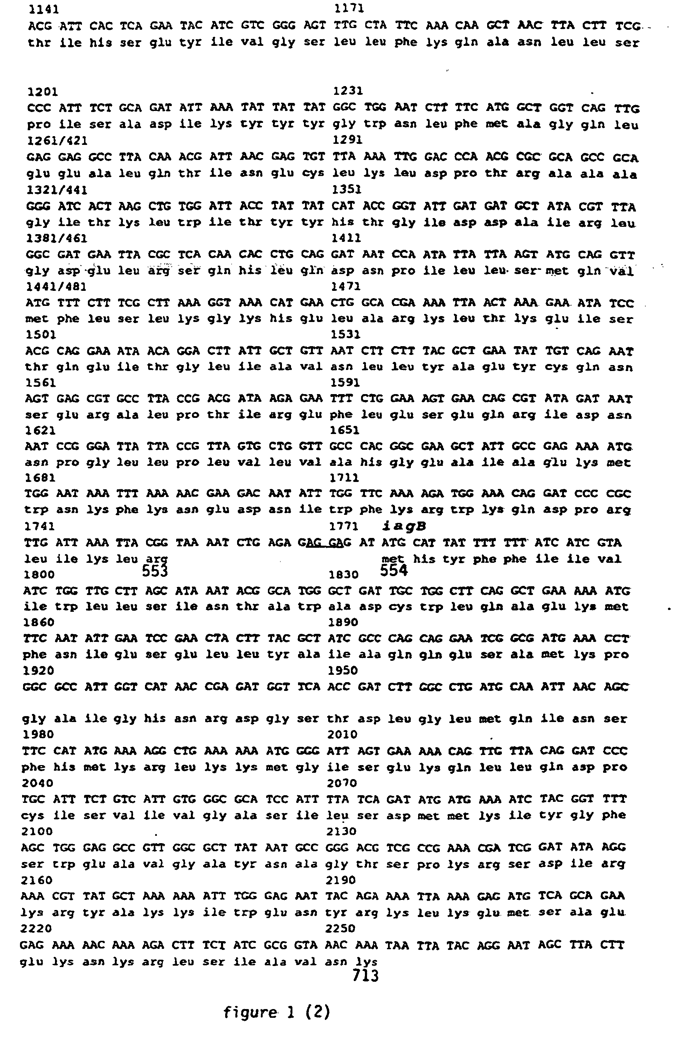

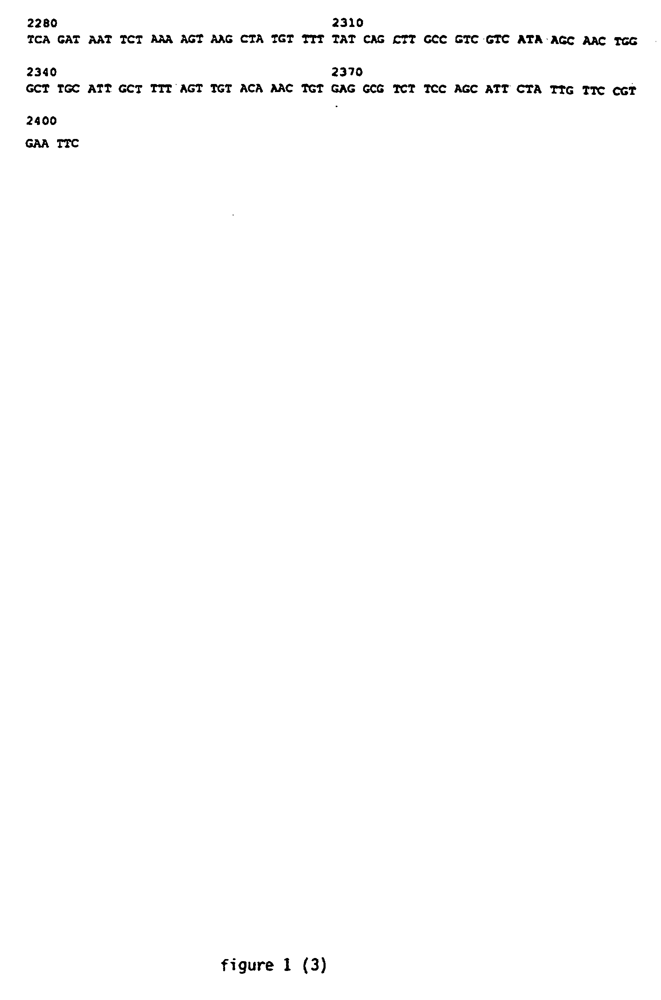

L'invention concerne aussi les séquences de nucléotides, participant à l'invasion de cellules par Salmonella enterica sous-espèce enterica sérovariété Typhi, caractérisées en ce qu'il s'agit de la séquence iagA comprise entre les nucléotides 97 et 1755 de la séquence représentée à la figure 1.

La présente demande a également pour objet la protéine IagA répondant aux séquences présentées à la figure 1.

De façon générale, l'invention a pour objet toute séquence d'acides aminés codée par le gène iagA représenté à la figure 1.

Le procédé d'infection des cellules HeLa en culture est le procédé standard qui a notamment été décrit dans la demande de brevet internationale publiée sous le numéro WO 92/01056.

Selon un autre aspect, l'invention concerne des moyens pour la détection de la présence de S. enterica et/ou S. bongori et le cas échéant, pour la quantification de S. enterica et/ou S. bongori dans des échantillons biologiques.

Par échantillon biologique, on entend tout échantillon prélevé pour la réalisation d'analyses in vitro chez l'animal ou chez l'homme ou prélevé à partir de produits alimentaires quelle qu'en soit la nature ou à partir de tout milieu liquide, solide ou gazeux susceptible de contenir les agents pathogènes recherchés.

Les conditions d'hybridation sont définies en fonction de la spécificité recherchée de l'hybridation et des conditions appropriées sont données à titre indicatif dans les exemples de la présente demande.

Des séquences de type oligonucléotides peuvent être sélectionnées pour être utilisées comme amorces soit pour la détection après amplification, de l'ADN génomique ou de l'ADNc de Salmonella de l'espèce S. enterica et/ou de l'espèce S. bongori appartenant aux autres groupes I à VI ou d'une partie de ces groupes soit dans d'autres conditions pour la détection spécifique de S. enterica du groupe I. Il peut s'agir notamment de séquences de nucléotides obtenues par synthèse chimique selon les métodes connues de l'homme du métier.

Des oligonucléotides préférés, utilisables pour l'amplification d'acide nucléique caractéristique de bactéries appartenant à l'un des groupes I, II, IIIa, IIIb, IV, V ou VI du genre Salmonella et notamment de l'ADN génomique ou de l'ADNc de S. enterica et/ou de S. bongori sont par exemple les suivants (leur position au sein de la séquence IagA représentée à la figure 1 étant indiquée) :

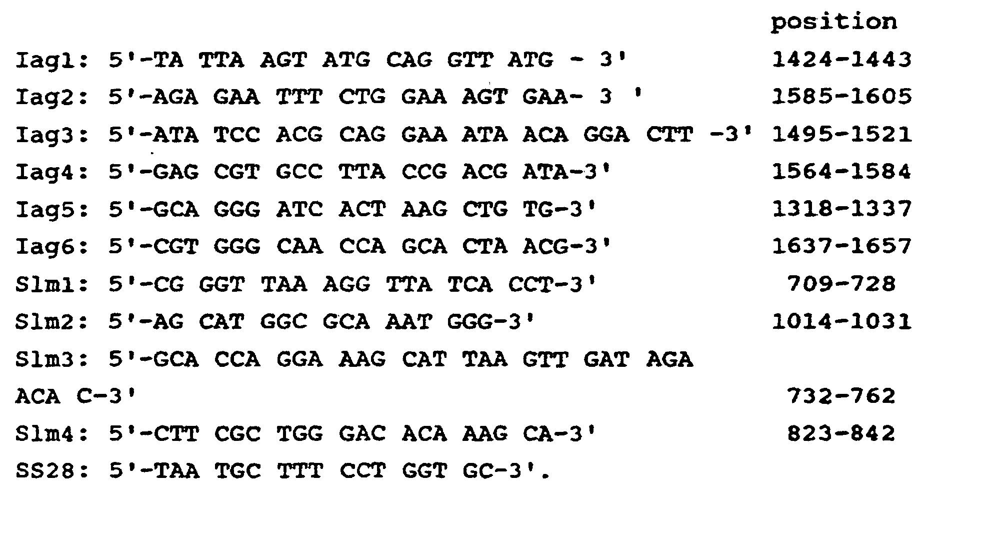

Ces oligonucléotides peuvent également être utilisés comme sondes, par exemple pour la détection des produits d'amplification de l'ADN et/ou de l'ADNc de S. enterica et/ou de S. bongori.

Un couple d'amorces préféré pour réaliser l'amplification de l'acide nucléique de S. enterica et/ou de S. bongori, quel que soit le groupe d'appartenance de la bactérie, est par exemple formé des amorces Iag5 (sens) et Iag6 (antisens).

Ce couple d'amorces dirige l'amplification d'un fragment d'acide nucléique de 340 bp.

Un autre couple d'amorces préféré est formé des amorces Slm1 (sens) et Slm2 (antisens). Ces amorces sont susceptibles de s'hybrider avec l'ADN ou l'ADNc de bactéries S. enterica et/ou de S. bongori de l'un des groupes I, II, III, IV, V ou VI.

Selon un autre mode de réalisation préféré, l'invention concerne des oligonucléotides utilisables comme amorces pour la détection spécifique de Salmonella enterica Groupe I lorsque les conditions de détection après l'amplification de l'ADN ou de l'ADNc sont celles qui sont décrites dans l'exemple 2.

De telles amorces sont caractérisées par leur capacité à amplifier des séquences d'acide nucléique des souches de S. enterica ou de S. bonqori représentatives des groupes I, II, III, IV, V et VI, mais pour lesquelles les conditions de détection sont celles exposées dans l'exemple I, permettant la seule détection des bactéries du groupe I.

Un couple d'oligonucléotides utilisables à ces fins, en tant qu'amorces spécifiques pour la détection de séquences d'ADN ou d'ADNc de S. enterica du groupe I est par exemple constitué par les séquences suivantes : SS2 5'-CCGGGCAGATGATACCC-3' et

SS28 '-TAATGCTTTCCTGGTGC-3'.

Les oligonucléotides définis par les inventeurs, permettent d'envisager le diagnostic de S. enterica et/ou de S. bongori dans des conditions satisfaisantes de sensibilité de rapidité, de facilité et de spécificité.

L'invention également a pour objet un kit pour la détection de S. enterica et/ou de S. bongori par amplification de l'ADN génomique ou complémentaire de S. enterica et/ou de S. bongori, caractérisé en ce qu'il comprend :
- des oligonucléotides iag3, iag4, iag5 et iag6, capables d'hybrider dans des conditions stringentes avec l'ADN génomique ou l'ADNc de S. enterica et/ou de S. bongori,
- une sonde, iag3 ou iag4, pour la détection des fragments amplifiés répondant à l'une des définitions données dans les pages précédentes,
- les réactifs nécessaires à la réalisation de la réaction d'amplification.

L'invention a donc en particulier pour objet, l'utilisation des oligonucléotides précités, comme amorces pour l'amplification d'une séquence d'ADN ou d'ADNc de Salmonella enterica et/ou de Salmonella bongori, comprise dans l'une des séquences iagA telles que décrites dans les pages précédentes ou complémentaires d'une telle séquence, ou encore l'utilisation de ces oligonucléotides comme sonde pour la détection d'une séquence de nucléotides amplifiée.

Par exemple, les oligonucléotides iag5 et iag6 peuvent être utilisés respectivement comme amorces sens et antisens pour la détection de S. enterica et/ou de S. bongori du groupe I, II, III, IV, V ou VI.

De même, le couple d'amorces Slml et Slm2 peut être utilisé pour la détection de bactéries de l'espèce S. enterica et/ou de S. bongori de l'un de ces groupes dans un échantillon biologique.

La détection est spécifique lorsque les amorces utilisées pour l'amplification des séquences de nucléotides recherchées permettent l'amplification de bactéries S. enterica et/ou de S. bongori appartenant à l'un des autres groupes II, III, IV, V ou VI, mais que les conditions mises en oeuvre ne permettent pas la détection des bactéries de ces mêmes groupes ou d'organismes différents susceptibles d'être présents dans l'échantillon biologique testé.

L'invention concerne ainsi un ensemble d'oligonucléotides utilisables pour la détection de bactéries S. enterica et/ou de S. bongori, après amplification de l'ADN génomique ou complémentaire de S. enterica et/ou de S. bongori, comme défini dans la revendication 7.

Un premier ensemble d'oligonucléotides utilisables pour la détection in vitro dans un échantillon biologique, de souches de Salmonella enterica et/ou de S. bongori appartenant à l'un des groupes I, II, III, IV, V ou VI, est caractérisé en ce qu'il contient les oligonucléotides suivants :
- la séquence Iag5 (5'-GCA GGG ATC ACT AAG CTG TG-3' et la séquence Iag6 (5'-CGT GGG CAA CCA GCA CTA ACG-3') utilisables en tant qu'amorces pour l'amplification et
- la séquence Iag3 (5'-ATA TCC ACG CAG GAA ATA ACA GGA CTT -3') utilisable comme sonde de révélation et la séquence Iag4 (5'-GAG CGT GCC TTA CCG ACG ATA-3') utilisable comme sonde de capture.

Un autre ensemble d'oligonucléotides utilisables pour la détection spécifique in vitro dans un échantillon biologique, de S. enterica du groupe I, est caractérisé en ce qu'il comprend les oligonucléotides suivants : SS2 (5'-CCGGGCAGATGATACCC-3' et

SS28 ('-TAATGCTTTCCTGGTGC-3').

La présente demande a par ailleurs pour objet une protéine iagA codée par la séquence de nucléotides iagA représentée à la figure 1.

De façon préférée, la protéine iagA a la séquence d'acides aminés représentée à la figure 1.

Entre également dans le cadre de l'invention un procédé pour la détection in vitro dans un échantillon biologique comme défini dans la revendication 11, de séquences de nucléotides Salmonella enterica et/ou de S. bongori, préalablement amplifiées par exemple par PCR caractérisé en ce qu'il comprend les étapes de :
- dénaturation de la séquence de S. enterica et/ou de S. bongori amplifiée,
- mise en contact des séquences de nucléotides amplifiées dénaturées de S. enterica et/ou de S. bongori, avec une sonde de capture et une sonde de révélation obtenues à partir des oligonucléotides ci-dessus définis dans des conditions permettant l'hybridation desdites sondes de capture et de révélation avec la susdite séquence de nucléotides amplifiée de S. enterica et/ou de S. bongori, la sonde de capture étant fixée à la surface d'un puits d'une plaque de microtitration et la sonde de révélation étant marquée et libre dans un tampon d'hybridation approprié ;
- incubation du mélange réactionnel, pendant un temps suffisant pour permettre la réaction d'hybridation ;
- lavage pour éliminer les oligonucléotides n'ayant pas réagi ;
- révélation des sondes de révélation ayant hybridé aux séquences de nucléotides amplifiées.

Le procédé de détection précédemment décrit peut être avantageusement caractérisé en ce que la détection est réalisée conformément aux étapes suivantes :
- dénaturation d'un volume 10 µl de la séquence amplifiée par addition volume à volume d'une solution 200 mM NaOH, 40mM EDTA,
- préhybridation des microplaques dont la surface des puits est revêtue de la sonde de capture, dans un tampon d'hybridation approprié,
- libération de la microplaque et remplissage de chacune des cupules avec 200µl de tampon d'hybridation renfermant le fragment amplifié dénaturé et la sonde de révélation marquée à la peroxydase à la concentration de 10ng/µl,
- incubation du mélange pendant une heure à 37°C sous agitation,
- lavage du mélange ayant réagi avec une solution de lavage 10X (100mM Tris, 3M NaCl, 1% Tween 20, pH 7,4),
- détection de l'activité de la peroxydase liée à la sonde par colorimétrie en présence d'un substrat coloré.

La révélation de l'activité de la peroxydase présente sur la sonde de révélation peut être obtenue par mise en oeuvre des étapes suivantes :
- dépôt de 200µl d'une solution 40mM citrate trisodique, 0,03% H₂O 30%, 7,5mg/ml d'orthophenylenediamine (OPD) dans chacun des puits contenant le mélange de réaction,
- incubation de la microplaque pendant 30min à l'obscurité et à 37°C,
- bloquage de la réaction par addition de 50µl/puits d'une solution 4N H₂SO₄,
- détermination de la densité optique à une longueur d'onde de 492nm (référence à 620nm).

De façon intéressante, la sonde de capture utilisée est l'oligonucléotide Iag4 et la sonde de révélation est l'oligonucléotide Iag3.

Ainsi, les moyens définis dans le cadre de l'invention permettent la détection qualitative ou quantitative de la présence de bactéries de type S. enterica et/ou de S. bongori, qu'il s'agisse d'une détection non spécifique au sein de l'un des groupes I, II, III, IV, V ou VI de S. enterica et/ou de S. bongori.

Dans des conditions spécifiques de mise en oeuvre de l'étape de détection, telles qu'exposées dans l'exemple I, les amorces SS2, SS28 et la sonde SS40 permettent au contraire la détection spécifique de bactéries du groupe I de S. enterica.

D'autres caractéristiques et avantages de l'invention apparaissent dans les exemples qui suivent et dans les figures :
Figure 1 : Séquence de nucléotide d'un fragment d'ADN de 2,4 kb de la région d'invasion de Salmonella ser. Typhi.
   Les sites potentiels de liaison au ribosome sont soulignés.
Figure 2 : Pourcentage de l'activité obtenue avec différentes souches de Salmonella appartenant à différentes sérovariétés par hybridation sandwich.
   Sérovariétés des différents isolats de Salmonella testés :
   a: S. enterica sous-espèce enterica (1), réf.: C53
   b: S. enterica sous espèce salamae (II), réf.: 975-71
   c: S. enterica sous espèce salamae (II), réf.: 3975-83
   d: S. enterica sous espèce arizonae (IIIa), réf.: 1600 K
   e: S. enterica sous espèce arizonae (IIIa), réf.: So 20-20
   f: S. enterica sous espèce diarizonae (II), réf.: 5250-85
   g: S. enterica sous espèce diarizonae (IIIb), réf.: 8013-93
   h: S. enterica sous espèce houtenae (IV), réf.: 1357-73
   i: S. bongori, ref.: 2790-79
   k: S. enterica sous espèce indica (VI), réf.: 4355-84 - 7, 6, 5, 4, et 3: log (montant des molécules d'ADN).

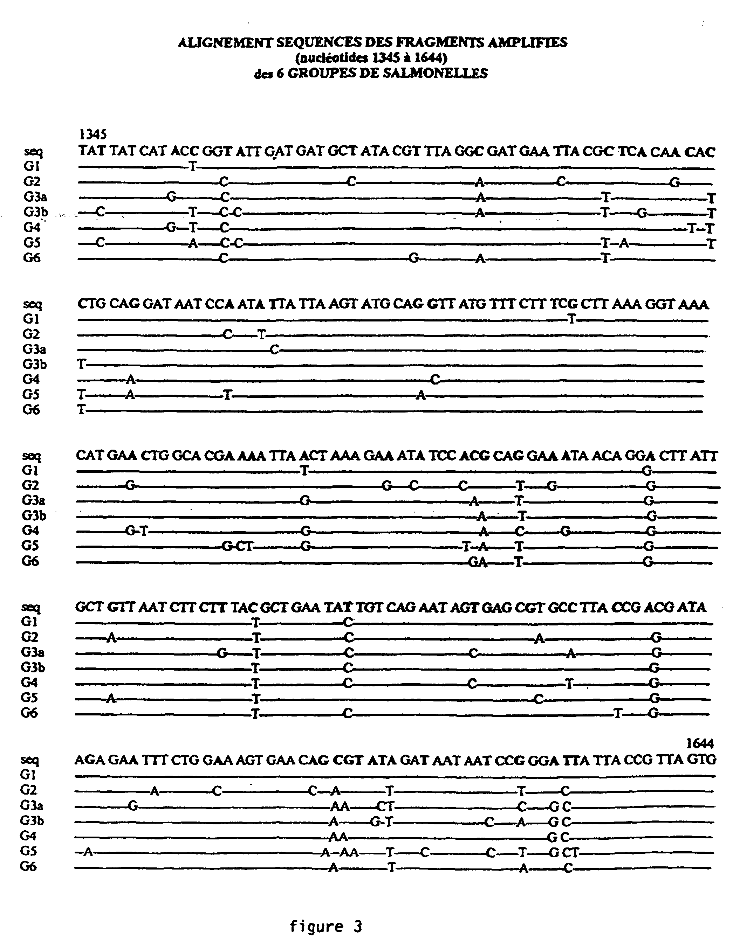

Figure 3 : Alignement des séquences des fragments amplifiés (nucléotides 1345 à 1644) des 6 groupes de Salmonelles.

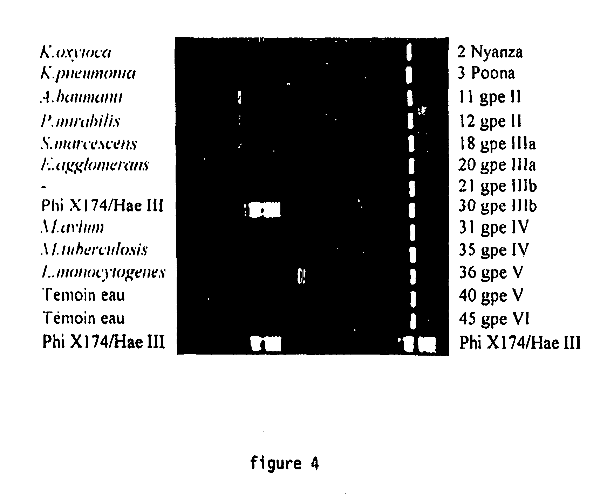

Figure 4 : Amplification grâce aux amorces Iag5 et Iag6 sur deux représentants de chacun des groupes de Salmonelles.

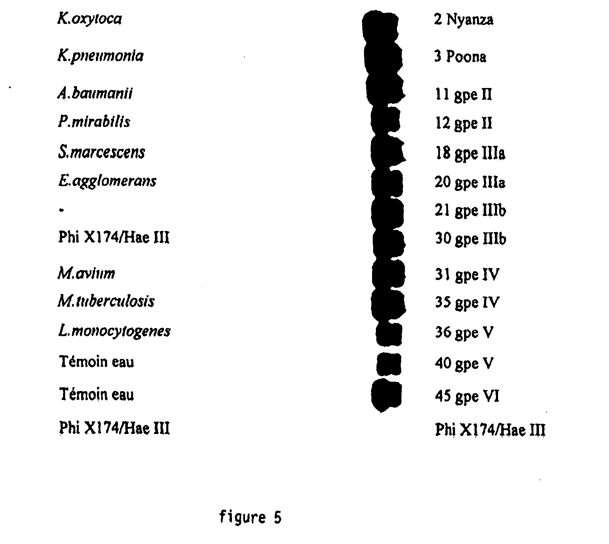

Figure 5 : Autoradiographie du Southern blot des produits amplifiés des Salmonelles.

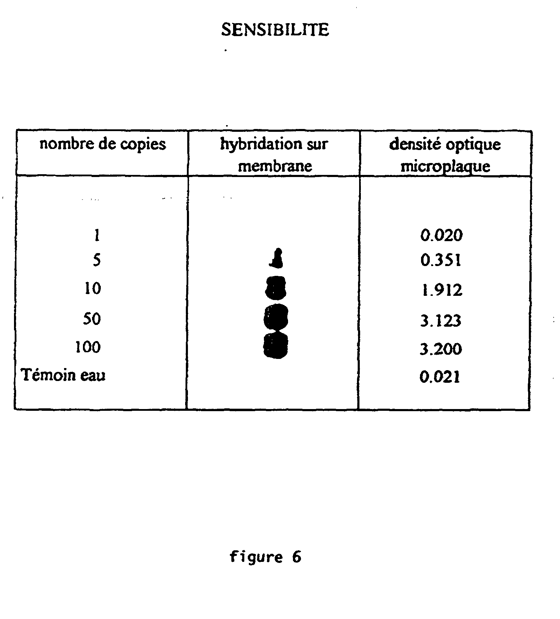

Figure 6 : Détermination du nombre minimal de molécules d'ADN chromosomique pouvant être détecté.
   Autoradiographie du Southern blot et hybridation sur microplaque.

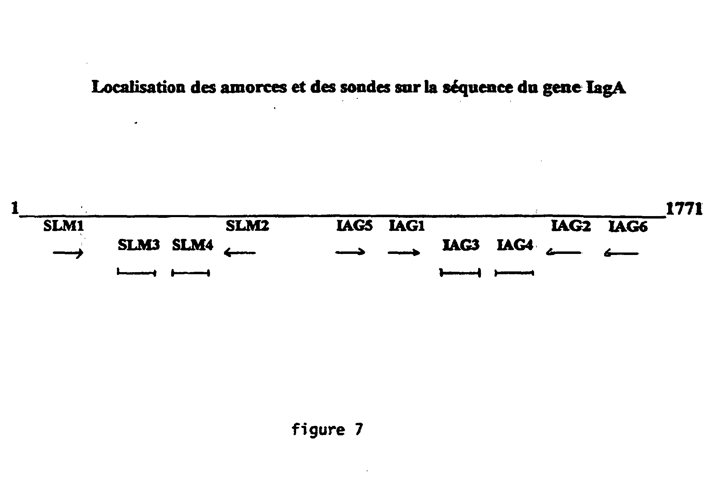

Figure 7 : Localisation des oligonucléotides sélectionnés au sein du gène IagA.

### EXEMPLE I

### CLONAGE ET SEQUENCAGE DU FRAGMENT D'ADN DE 2,4 kb

Ce fragment d'ADN a été sous-cloné en utilisant un fragment de restriction obtenu par coupure avec les enzymes HindIII, à partir de la séquence HindIII de 7,9 kb décrite dans la publication de Popoff et Dion, 1990, dans des dérivés du vecteur m13 (Messing et Vieira, 1982).

Après la réalisation de ce clonage, la méthode de dideoxy terminaison de chaine a été mise en oeuvre en utilisant la T7 DNA polymérase modifiée (Sequenase, USB Corp.) et des oligonucléotides synthétiques universels à titre d'amorces. Toutes les extrémités des fragments de restriction utilisés se chevauchaient les unes les autres. Le séquençage de l'ADN a été réalisé au moins 2 fois sur chacun des brins. La séquence de nucléotides a été analysée en utilisant le programme de Lipan et Pearson, 1985.

Comme le montre la séquence présentée à la figure 1, deux phases ouvertes de lecture sont contenues dans le fragment séquencé ; elles sont désignées par les termes iagA (abréviation de invasion associate gene) et iagB. Les deux phases ouvertes de lecture sont transcrites dans la même orientation. Le premier codon ATG (bp 97) de la phase ouverte de lecture de l'iagA, qui est précédé par la séquence 5'-AGAGA-3', est supposé correspondre au site d'initiation de la traduction du gène iagA. Le gène iagA code pour un polypeptide comportant 553 résidus d'acides aminés avec un poids moléculaire calculé de 63026 Da. Une homologie significative a été détectée entre le domaine N-terminal de la protéine IagA et le domaine correspondant à la protéine de régulation de la transcription PhoB (24% d'identité et 52% de similitude pour une superposition de 108 acides aminés) et la protéine PhoP (25% d'identité et 69% de similitude pour 100 acides aminés alignés) de E.coli. Le codon d'initiation ATG du gène iagB (bp 1776) est également précédé par un site potentiel de liaison au ribosome (5'-AGGAAG-3'). Le gène iagB code pour un polypeptide comportant 160 acides aminés et ayant un poids moléculaire calculé de 18369 Da. La comparaison de la séquence de la protéine IagB avec les séquences traduites contenues dans la banque de données Genbank a montré une homologie significative avec la protéine IpgF (43% d'identité et 66% de similitude pour 151 acides aminés alignés).

La protéine IpgF est codée par le gène ipgF qui est situé sur le plasmide associé à la virulence de Shigella flexneri, à l'extrémité 5' du locus mxi-spa (Allaoui et al, 1993).

Les protéines mises en évidence de Salmonella enterica sous-espèce enterica sérovariété Typhi auraient donc un rôle dans l'infection par ces bactéries, et notamment dans l'adhésion et la pénétration dans les cellules.

### EXEMPLE 2

### DETECTION SPECIFIQUE DE S. ENTERICA DU GROUPE I

Un protocole de détection des sous-espèces de Salmonella par réaction de polymérisation en chaîne (PCR) a été mis au point. Un couple d'oligonucléotides utilisés comme amorce a été défini pour amplifier un fragment de 93 pb d'un gène requis pour l'invasion des cellules HeLa par S. typhi, souche Ty2. Le produit d'amplification a été analysé par une hybridation non radioactive en sandwich sur des plaques de microtitration en utilisant deux oligonucléotides différents selon la procédure décrite par Chevrier et al, 1993, Mol. Cell. Probes 7, 187-197. L'oligonucléotide de capture a été phosphorylé à son extrémité 5' et lié de façon covalente à des puits portant des groupes aminés d'une plaque de microtitration. L'oligonucléotide de détection a été aminé à son extrémité 5' puis marqué avec un biotinyl-N-hydroxy-succinimide ester. Après hybridation, les molécules hybrides ont été détectées par de l'avidine conjuguée à la phosphatase alkaline et à un substrat chromogène. Cette méthode nécessite seulement l'utilisation d'un cycler thermique et d'un lecteur de microtitration conventionnel, et peut être mise en oeuvre sur une large échelle.

### MATERIELS ET METHODES

### Souches bactériennes

Deux cent vingt huit isolats cliniques (Tableau 1) incluant S. bongori (Sambrook et al, 1989, Molecular cloning, a laboratory manual. Cold Spring Harbor Laboratory Press, Cold Spring Harbor, N.Y.), S. enterica sous espèce I(116), II(56), IIIa(11), IIIb(30), IV(5) et VI(5) et 16 souches d'Enterobactéries non-salmonelles (Tableau 2) représentant 9 genres différents ont été utilisés dans cette étude. La souche C53 de S. ser. Typhimurium a été utilisée comme contrôle positif, et la souche HB101 de E. coli a été utilisée comme contrôle négatif dans les tests de PCR.

### Extraction de l'ADN

Les souches ont été cultivées sur un milieu LB à 37°C. Afin de procéder à l'extraction rapide de l'ADN, 2 ml de la culture maintenue pendant toute la nuit ont été centrifugés et resuspendus dans 1 ml de TE (tampon 10 mM Tris-HCl à pH8 contenant 1 mM EDTA). Les cellules ont été centrifugées, le culot de centrifugation a été resuspendu dans 500 µl d'eau distillée stérile et chauffé à 100 °C pendant 10 minutes. Finalement, la solution a été centrifugée et le surnageant a été conservé pour des expérimentations en PCR.

### Amorces oligonucléotidiques et sondes

Les oligonucléotides ont été synthétisés dans un synthétiseur à ADN cyclone (Millipore-Waters) en utilisant la technologie au phosphoramidite.

Les séquences des amorces d'oligonucléotides étaient les suivantes : SS2: 5'-CCGGGCAGATGATACCC-3' et

SS28: 5'-TAATGCTTTCCTGGTGC-3'.

La sonde oligonucléotidique de capture, SS40: 5'-CCCGAACTATCTCGATCTGTACAATATTATCATT-3' a été phosphorylée à son extrémité 5' avec la T4 polynucléotide kinase (Boehringer) selon la description faite par Sambrook et al, 1989, (Molecular cloning, a laboratory manual. Cold Spring Harbor Laboratory Press, Cold Spring Harbor, N.Y.). La sonde de détection octadecanucléotidique SS41 (5'-GCAGGTGATAACCTTTAA-3') a été synthétisée avec une fonction amino à son extrémité 5' en utilisant la méthode au phosphoramidite sur phase solide dans un synthétiseur d'ADN Applied Biosystem 380B puis marquée avec le D-biotinyl-Σ-aminocaproic acide-N-hydroxysuccinimide ester (Boehringer) selon la description faite par Tham et al, 1990, (FEMS Microbiol. Lett. 69, 109-116). Les oligonucléotides de capture et de détection ont été tous les deux purifiés sur une colonne de dessalement rapide HR 10/10 avec le système FPLC (Pharmacia).

### Expériences de PCR

Les réactions d'amplification ont été réalisées dans un volume total de 100 µl dans un tampon 50 mM Tris-HCl pH 8,5, contenant 4 mM MgCl₂, 100 µg/ml de sérum alumine bovine, 1µM de chaque amorce, 200 µM de chaque dNTP et 1 U de Taq ADN polymérase (Amersham). Le mélange d'amplification a été recouvert avec 100 µl d'huile minérale et soumis à 10 cycles d'amplification selon la description suivante : les échantillons ont été incubés à 94°C pendant 10 sec pour dénaturer l'ADN, à 60°C pendant 10 secondes pour apparier les amorces à l'ADN et à 72°C pendant 30 sec. pour réaliser la réaction d'extension des amorces appariées, ceci étant suivi de 30 cycles selon le protocole suivant : dénaturation à 87°C pendant 10 sec., appariement à 60°C pendant 10 sec. et extension à 72° C pendant 30 sec. Les cycles thermiques ont été réalisés dans un ensemble chauffant programmable (Thermal ractor Hybaid, UK).

Les expériences de PCR ont été réalisées avec 5µl de solution d'ADN. Chaque expérience comprenait des contrôles négatifs (5 µl de tampon TE) pour chaque groupe de 10 échantillons et à la fin de chaque série.

### Tests d'hybridation sandwich sur des bandes CovaLink NH®

Deux protocoles d'hybridation non-radioactive sur des bandes CovaLink NH® ont déjà été décrits par Chevrier et al (1993, Mol. Cell. Probes 7, 187-197). Dans le cas présent, une technique d'hybridation sandwich a été utilisée dans la mesure où elle permettait une meilleure sensibilité de la détection. La réaction a été réalisée sur des micropuits recouverts par de l'oligonucléotide SS40 en utilisant une procédure de liaison covalente telle que décrite en détails par Chevrier et al ou Rasmussen, S.R. et al (1991, Anal. Biochem. 198, 138-142).

Le fragment d'ADN soumis à la réaction de PCR a été dénaturé directement dans les puits en ajoutant de façon séquentielle 95 µl d'eau distillée, 5µl d'échantillon de PCR, 40 µl de sonde de détection et 14 µl de NaOH 1 N par puits. Après 10 minutes, la neutralisation a été effectuée en ajoutant 21 µl de NaH₂PO₄ 1 M contenant 1% de sarkosyle. Tous les échantillons ont été réalisés en double. Après la neutralisation, la bande a été déposée sur une surface métallique et maintenue dans un four pendant la nuit à 40°C. La concentration finale de la sonde de détection biotinylée SS41 était 0,5 nM. Pendant l'incubation dans le four, il est préférable de ne pas laisser les puits non utilisés vides, mais de les remplir avec de l'eau de façon à obtenir des échanges thermiques homogènes. Les micropuits ont été lavés 5 fois à température ambiante avec du TBS-Tw (0,15 M NaCl, 10 mM tampon Tris HCl à pH 8, 1%. Tween 20). 100 µl de conjugué alkaline phosphatase-extravidine (Sigma) dilués à 1 µg/ml dans du TBS-Tw contenant 1% de sérum albumine bovine ont été ajoutés par puits. Puis, la bande a été incubée à température ambiante pendant 1 h., lavée 5 fois avec du TBS-Tw et finalement 200 µl de diéthanolamine 1 M à pH 9,8 contenant 1mM de MgCl₂ et 1 mM de para-nitrophényl phosphate ont été ajoutés. La réaction de l'enzyme a été conduite pendant 30 minutes à 2 heures. L'absorbance a été mesurée à 405 nm en utilisant un lecteur de microplaque (Dynatech). Le signal obtenu avec la solution standard du fragment d'ADN amplifié (800 fm/puits) de S. ser. Typhimurium souche C53 a été considéré comme représentant 100% et utilisé comme référence pour chaque test d'hybridation. Les valeurs des blancs correspondent à l'absorbance moyenne mesurée dans des puits recouverts par l'oligonucléotide SS40 incubé seulement avec 0,5 nM de sonde oligonucléotidique SS41 biotinylée.

### RESULTATS

### Optimisation de la méthode

Les amorces et les sondes ont été choisies dans la séquence iagA. Différents couples d'amorces ont été testés pour optimiser la technique d'hybridation sandwich sur des microplaques CovaLink. Le couple d'amorces choisi (SS2 et SS28) a permis l'amplification spécifique de la région de 93 pb de l'ADN génomique de Salmonella. En utilisant ce couple d'amorces, on a mis en évidence qu'une concentration standard de MgCl₂ (1,5-2 mM) a conduit à un résultat d'amplification relativement inintéressant et qu'une concentration de 4 mM en MgCl₂ était nécessaire pour obtenir une amplification efficace. Des oligonucléotides internes, SS40 et SS41, ont été utilisés dans un test d'hybridation non radioactif à titre de sonde de capture et de sonde de détection respectivement.

### Spécificité de la technique

La spécificité de la méthode pour la détection des salmonelles a été évaluée avec 228 souches de Salmonella (Tableau 1) et 16 souches de bactéries hétérologues (Tableau 2). Les résulats sont résumés dans le tableau 3. Edwardsiella tarda, Klebsiella pneumoniae, des espèces d'Enterobacter et d'Acinetobacter, Pasteurella, Vibrio harveyi, Serratia marcescens et de façon plus importante des espèces de Citrobacter et tous les E. coli ont donné un signal d'hybridation inférieur à 20%. Sur la base de cette valeur, il a été conclu que toutes les souches de Salmonella appartenant à la sous-espèce I pouvaient être détectées par la présente méthode. De plus, seulement une souche (souche 3975-83) des 56 souches de la sous-espèceII et 3 souches des 11 souches de la sous-espèce IIIa ont donné un signal positif. Salmonella bongori et les souches appartenant aux sous-espèces IIIb, IV et VI n'étaient pas détectables.

### Niveau de détection de la technique avec les bactéries entières

Des dilutions au 1/10ème d'une suspension de la souche C53 de S. ser. Typhimurium (de 10⁹ à 10⁻² cellules/ml) ont été faites pour estimer le nombre minimum de bactéries qui pouvait être détecté par la PCR suivi de la technique d'hybridation non radioactive. L'ADN a été extrait de chaque suspension calibrée, en utilisant la technique d'extraction rapide par ébullition. Les résultats obtenus montrent clairement que la technique d'extraction rapide de l'ADN par la simple ébullition de la suspension avant la réaction de PCR, est une technique efficace. En effet, elle permet la détection de seulement une unité cfu.

**Tableau 1 :**

| Sous-espèces de Salmonella utilisées pour évaluer la spécificité des essais d'hybridation de l'ADN. | | |
|---|---|---|
| Microorganisme testé | N° des isolats | N° des serovars |
| *Salmonella enterica* subsp *enterica* I | 116 | 43 |
| serovar Adelaïde | | 1 |
| Agona | | 2 |
| Altona | | 1 |
| Angoda | | 1 |
| Bardo | | 2 |
| Blockley | | 1 |
| Bovismorbificans | | 3 |
| Braenderup | | 4 |
| Brandenburg | | 1 |
| Bredeney | | 1 |
| Broughton | | 2 |
| Cerro | | 1 |
| Chester | | 1 |
| Coeln | | 1 |
| Concord | | 1 |
| Dakar | | 1 |
| Derby | | 2 |
| Enteridis | | 28 |
| Georgia | | 1 |
| Hadar | | 1 |
| Heidelberg | | 4 |
| Ibadan | | 2 |
| Indiana | | 1 |
| Infantis | | 5 |
| Lexington | | 1 |
| London | | 1 |
| Mbandaka | | 1 |
| Montevideo | | 6 |
| Moscow | | 1 |
| Ohio | | 1 |
| Orion | | 1 |
| Panama | | 3 |
| Paratyphi B | | 2 |
| Saintpaul | | 1 |
| Typhimurium | | 13 |
| Typhisuis | | 1 |
| Vaertan | | 1 |
| Veneziana | | 1 |
| Vinohrady | | 1 |
| Virchow | | 10 |
| Wien | | 1 |
| Woodinville | | 1 |
| Yolo | | 1 |
| *Salmonella enterica* subsp *salamae* II | 56 | 56 |
| *Salmonella enterica* subsp *arizonae* IIIa | 11 | 29 |
| *Salmonella enterica* subsp *diarizonae* IIIb | 30 | 5 |
| *Salmonella enterica* subsp *houtenae* IV | 5 | 5 |
| *Salmonella enterica* subsp *indica* VI | 5 | 5 |
| *Salmonella bongori* | 5 | 5 |
| (initialement *S. enterica* subsp. *bongori* V) | | |

**Tableau 2 :**

| Bactéries hétérologues utilisées dans le test d'hybridation de l'ADN | | |
|---|---|---|
| Genre | Espèces | Nombre d'isolats |
| *Escherichia* | *coli* | 4 |
| *Edwarsiella* | *tarda* | 1 |
| *Citrobacter* | *amalonaticus* | 1 |
| | *freundii* | 1 |
| *Klebsiella* | *pneumoniae* | 1 |
| *Enterobacter* | *agglomerans* | 1 |
| | *asburiae* | 1 |
| | *hormoechei* | 1 |
| *Pasteurella* | *multocida* | 1 |
| *Acinetobacter* | *Iwoffii* | 1 |
| | *haemolyticus* | 1 |
| *Vibrio* | *harveyi* | 1 |
| *Serratia* | *marcescens* | 1 |

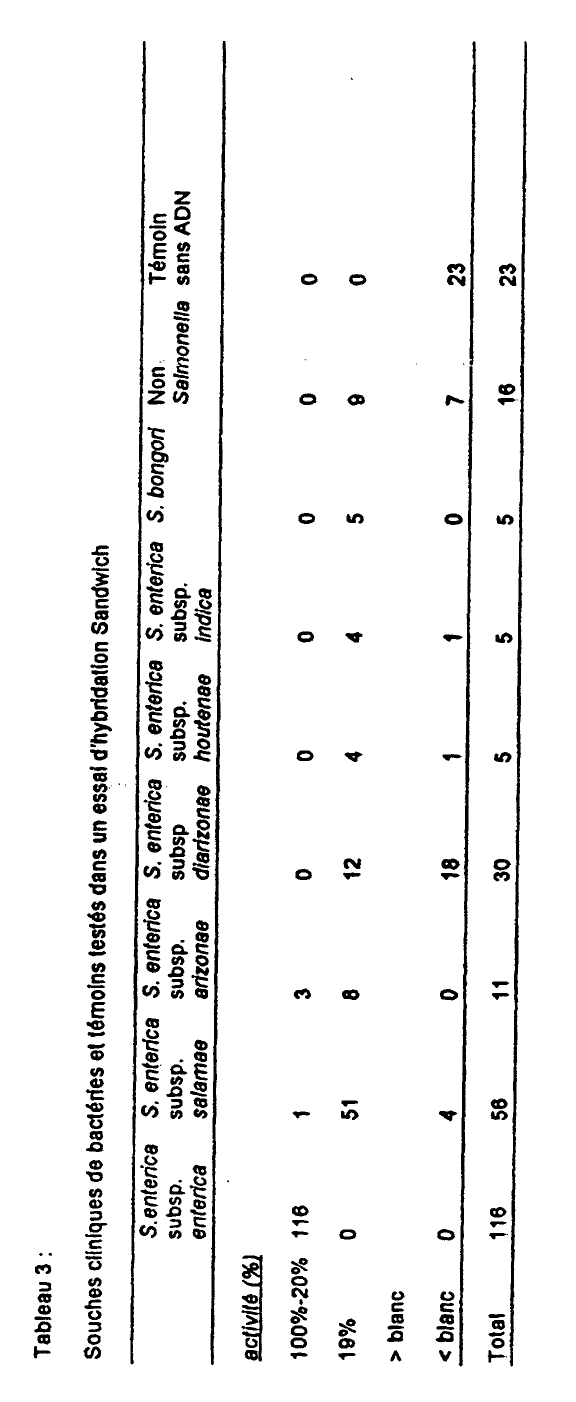

### Hybridation quantitative avec l'ADN génomique purifié

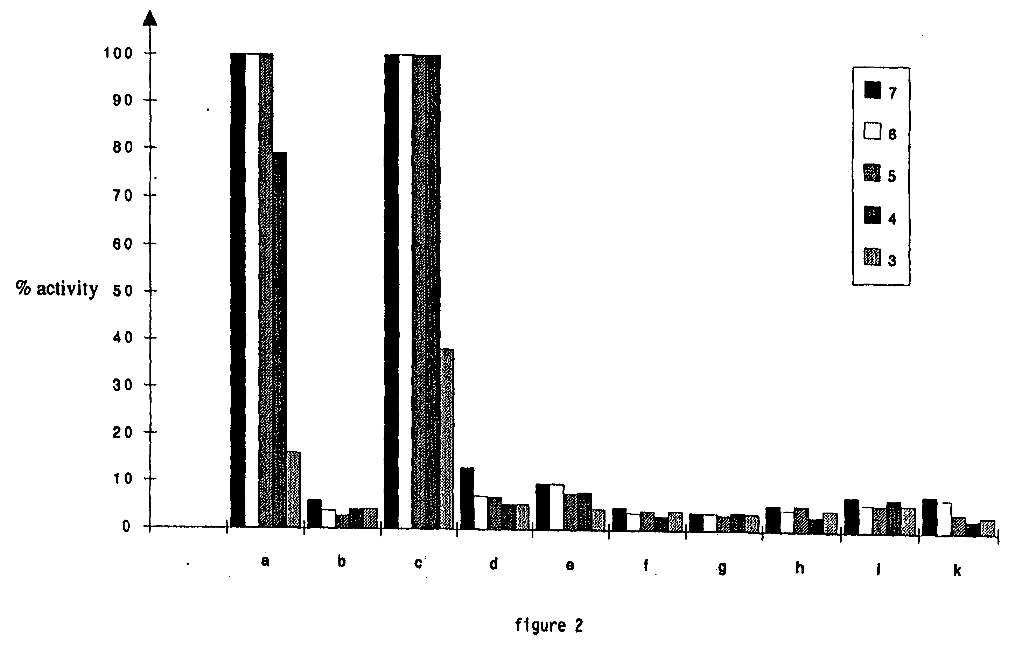

La procédure d'hybridation non-radioactive utilisée dans les tests rapportés ici peut être facilement mise en oeuvre dans des études quantitatives. Pour comparer les signaux d'hybridation obtenus avec différentes souches de Salmonella, l'ADN a été extrait de 10 souches représentant les 6 sous-espèces de Salmonella enterica et l'espèce Salmonella bongori, puis des quantités calibrées d'ADN ont été soumises à des réactions de PCR suivies par une hybridation sandwich. Les résultats sont rapportés à la figure 2. Il a été démontré que le signal d'hybridation obtenu avec 10⁷ molécules d'ADN de Salmonella bongori ou des sous-espèces II, IIIa, IIIb, IV et VI de Salmonella enterica est plus faible que le signal d'hybridation observé avec 10³ molécules d'ADN des souches de la sous-espèce I. Cependant, il est important de noter que l'isolat 3975-83 (sous-espèce II) a donné le même signal d'hybridation que les souches appartenant aux sous-espèces I.

### DISCUSSION

L'amplification par PCR permet une détection très sensible de séquences d'ADN spécifique. La sensibilité de l'amplification dépend essentiellement du nombre de copies de l'ADN cible, de la pureté de l'échantillon à analyser, de la méthode d'extraction de l'ADN, et de la sensibilité de la méthode utilisée pour détecter les produits de PCR. La visualisation des produits de PCR par une coloration au bromure d'éthidium dans un gel d'électrophorèse, n'est pas compatible avec l'utilisation en routine de la technique et n'est pas suffisamment sensible. La sensibilité peut être améliorée par l'utilisation de la PCR double ou de sondes d'ADN avec une hybridation en Dot blot ou en Southern blot. Cependant la PCR double est très sensible à la contamination par de l'ADN et les techniques d'hybridation Dot blot ou Southern blot ne sont pas appropriées pour l'automatisation. L'hybridation sur microplaque offre donc une technique appropriée pour la détection et la quantification de fragments amplifiés par PCR. L'attachement covalent simple des acides nucléiques à des micropuits représente une variante intéressante à l'adsorption passive et une amélioration importante pour la détection de fragments amplifiés par PCR sur des micropuits.

Il est connu que les souches de Salmonella provoquant des infections chez l'homme appartiennent essentiellement à la sous-espèce I. En effet, plus de 95% des isolats cliniques chez l'humain appartiennent à cette sous-espèce (Rowe, B., 1987, Salmonella surveillance. Reports received from centers participating in the WHO programme. World Health Organization London). De plus, en 1991, le "Centre National d'Etudes Vétérinaires et Alimentaires" de Paris (France) rapportait [Corbion, B. et al, 1991, Inventory of Salmonella] que dans les années précédentes, la plupart des souches isolées chez l'animal dans la nourriture ou dans l'environnement en 1988 et 1989 (c'est à dire, 18832 souches) appartient à la sous-espèce I (99,2%).

Les résultats rapportés ici ont permis de définir une méthode fondée sur l'amplification par PCR pour la détection de souches pathogènes de Salmonella. Un couple d'amorces, SS2 et SS28, et un couple de sondes, SS40 et SS41 ont été sélectionnés à partir d'un gène nécessaire à l'invasion des cellules HeLa par Salmonella ser. Typhi souche Ty2. En utilisant la combinaison entre la technique de PCR et l'hybridation sandwich non radioactive sur microplaque, toutes les Salmonella de la sous-espèce I ont été détectées.

La limite de détection était inférieure à un seuil représenté par 10 cellules par tube de PCR, ce qui est conforme aux résultats obtenus par d'autres techniques de PCR similaires. Etant donné la parenté des acides nucléiques entre les membres des enterobactéries, il était important de contrôler la spécificité de ces nouvelles amorces et sondes avec les genres d'enterobactéries les plus susceptibles de conduire à des réactions de type "faux-positif". Des résultats obtenus, on peut conclure qu'aucune réaction de faux-positif ne peut avoir lieu lorsque les conditions de la PCR et de l'hybridation décrites précédemment sont suivies.

Il est intéressant de noter que la souche Salmonella 3975-83 (sous-espèce II) présentait un signal d'hybridation identique à celui obtenu avec les isolats appartenant à la sous-espèce I. Cette souche a été isolée en 1983 à partir des selles d'un patient humain en Grande-Bretagne. Sur la base des caractéristiques biochimiques, cette nouvelle sérovariété a été classée dans la sous-espèce II mais a été considérée comme une souche atypique puisque sa présence dans la gélatinase n'a pas été détectée (Le Minor, L. et al, 1984, Supplement No. XXVII, 1983, to Kauffmann-White Scheme, Ann. Microbiol. (Institut Pasteur) 135 B, 45-51). A la lumière des résultats rapportés ici, la position taxonomique de la souche 3975-83 devrait être ré-examinée en utilisant la technique d'hybridation ADN-ADN.

Les données présentées ici indiquent que la méthode d'hybridation basée sur l'utilisation d'un gène nécessaire à l'invasion des cellules HeLa par Salmonella ser. Typhi souche Ty2 peut distinguer les souches de la sous-espèce I des Salmonella des autres bactéries enteriques, y compris E. coli. L'hybridation non-radioactive sur une microplaque Covalink NH est sensible et appropriée pour l'analyse d'un grand nombre d'échantillons.

### EXEMPLE 3 : DETECTION DE L'ADN DE SALMONELLA AMPLIFIE PAR HYBRIDATION SANDWICH

### Séquence des oligonucléotides

Les fragments d'ADN choisis sont les suivants (voir position sur la séquence de la figure 1) :

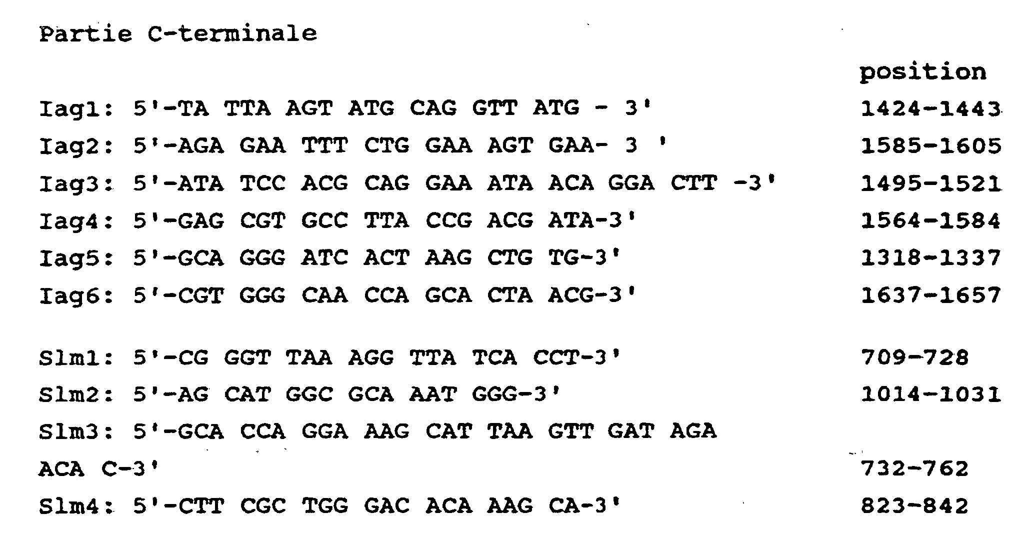

Préférentiellement, le couple d'amorces Iag5 (sens) et Iag6 (antisens) dirige l'amplification d'un fragment de 340bp, le couple Slm1 (sens) et Slm2 (antisens) dirige l'amplification d'un fragment de 323bp (figure 3).

La figure 4 montre l'efficacité de l'amplification du couple d'amorces Iag5 et Iag6 sur 2 représentants de chacun des groupes de Salmonelles.

### Procédé de détection.

Un format de détection par hybridation sandwich a été utilisé.

Deux oligonucléotides s'hybrident simultanément au fragment amplifié dénaturé. L'un d'eux appelé sonde de capture est fixé de façon passive (mais peut aussi être fixé de façon covalente) à la surface d'un puits de plaque de microtitration 96 puits. L'autre appelé sonde de révélation est marqué par un élément facile à mettre en évidence. La sonde de révélation est libre dans le tampon d'hybridation.

Les sondes de capture et de révélation sont complémentaires de 2 régions différentes situées à l'intérieur du fragment amplifié.

La sonde de détection dans le cas ici décrit, est liée à un marqueur enzymatique notamment une peroxydase et va servir de sonde de révélation. C'est le cas préférentiellement des oligonucléoties Iag3 et Slm3. D'autres oligonucléotides peuvent être fixés à un support solide de type microplaque, un support particulaire ou membranaire et servir de sonde de capture, ceci particulièrement pour les oligonucléotides Iag4 et Slm4.

### Conditions expérimentales:

### 1) Préparation de l'ADN des Salmonelles

Par la méthode d'ébullition en présence de Chelex (Chelex 6%, SDS 0.1%, NP40 1%, Tween 20 1%), on obtient les séquences d'ADN. Ce réactif est commercialisé par Biorad et utilisé selon le protocole du fabricant (ref. Walsh et al. 1991. BioTechniques 10 : 506-513).

### 2) Amplification

Selon la méthode initialement décrite par Saiki et telle qu'exposée par exemple dans le brevet européen EP 0201184.

La PCR est réalisée en utilisant le mélange réactionnel suivant :
50 mM KCl
10mM Tris-HCl pH 8.3
1,5mM MgCl₂
125 µM désoxyribonucléotides (dCTP, dATP, dGTP)
250 µM d'UTP
25 pmoles de chacune des amorces
10ng ADN
1 unité d'Uracyl N Glycosylase
1 unité de Taq polymerase.

Le mélange réactionnel a été réalisé en utilisant 10µl de la solution contenant l'ADN à amplifier sous un volume de 100µl. Le dUTP et l'UNG sont utilisés en système de décontamination (Brevet Life Technologies European Patent Application 0 401 037). Le thermocycler utilisé est le 9600 de Perkin Elmer.

Après une incubation à 50°C pendant 2 min pour permettre l'action de l'UNG et une dénaturation à 95°C pendant 5min, les cycles de température utilisés sont les suivants :
- 5 cycles (95°C 15 sec, 50°C 15 sec, 72°C 15 sec)
- 35 cycles (95°C 15 sec, 57°C 15 sec, 72°C 15 sec)

### 3) Visualisation de la réaction d'amplification

### 3-1) Marquage de la sonde de révélation

Les sondes sont marquées à la peroxydase de raifort (ref. PCR protocols : a guide to methodes and application; Academic press (1990), 15, p4513-4534) et l'activité de l'enzyme est révélée en colorimétrie.

### 3-2) Gel d'agarose coloré au BET et hybridation sur membrane

Après amplification, 10µl du produit d'amplification sont déposés sur gel d'agarose et l'ADN est transféré sur membrane selon les techniques classiques (Maniatis). La membrane est préhybridée, 30 min à 68°C en tampon d'hybridation (10X Denhart, 6X SSC, 0,1%SDS) puis hybridée à 42°C pendant 3h avec 60 ng de sonde par ml de tampon d'hybridation.

Un lavage est ensuite effectué selon les étapes suivantes :
- 2 fois 10 min en 2 X SSC -0,1%SDS à température ambiante,
- 1 fois 30 min en 0,1 X SSC - 0,1% SDS à 42°C,
- 2 fois 10 min en 2 X SSC à température ambiante.

Révélation : La membrane est épongée entre deux feuilles de papier absorbant (papier Whatman 3MM) et posée dans un bac propre et sec.

Le réactif de détection Amersham (réactif de détection ECL RPN 2105) est préparé extemporanément volume à volume ; 30 ml de volume total pour une membrane 5 x 8 cm. Une cassette pour autoradiographie est obtenue en fixant une feuille de papier absorbant (papier Whatman 3MM) au fond. Toutes ces étapes peuvent se faire à la lumière. Ensuite en chambre noire.

On baigne la membrane dans le réactif de détection pendant 1 min, côté ADN vers le haut, on égoutte rapidement la membrane, on la place dans la cassette, côté ADN vers le haut, on pose dessus une feuille en plastique transparent (sinon la membrane se colle sur le film) et on met un film radiographique par dessus (film X-OMAT KODAK).

L'exposition est réalisée pendant 30 min à température ambiante puis le film est développé par les techniques de révélation classique (révélateur, eau, fixateur).

### 3-3) microplaque

### 3-3-1) Coating de l'oligonucléotide de capture

Il peut se faire par adsorption (Cook et al, NAR, 16: 4077-4095 (1988) ou couplage covalent (Rasmussen, S.R. et al, 1991. Analytical Biochemistry 198, 138-142).

### 3-3-2)Hybridation en microplaque et lecture

10 µl du produit d'amplification ont été dénaturés par addition volume à volume d'une solution 200mM NaOH, 40mM EDTA.

Les microplaques dont la surface des puits est revêtue de la sonde de capture, ont été préhybridées dans un tampon d'hybridation contenant 10XDenhart, 6XSSC, 0,1% SDS.

Puis la microplaque a été vidée et chacune des cupules a reçu 200µl de tampon d'hybridation renfermant le fragment amplifié dénaturé et la sonde de révélation à la concentration de 10ng/µl. L'incubation a eu lieu pendant une heure à 37°C et sous agitation.

Après lavage (solution de lavage 10X :100mM Tris, 3M NaCl, 1% Tween 20, pH 7,4), l'activité de la peroxydase liée à la sonde a été détectée en colorimétrie en présence d'un substrat coloré.

Pour ce faire, 200µl d'une solution 40mM citrate trisodique, 0,03% H₂O 30%, 7,5mg/ml d'orthophenylenediamine (OPD) ont été distribués dans chacun des puits. La microplaque a été incubée 30min à l'obscurité et à 37°C. 50µl/puits d'une solution 4N H₂SO₄ ont été ajoutés pour bloquer la réaction.

La densité optique a été déterminée à une longueur d'onde de 492nm (référence à 620nm).

### 4) Séquençage des produits PCR et alignement manuel des séquences.

Selon les techniques classiques, en utilisant par exemple un automate "373 DNA sequencer" d'Applied Biosystem et le kit "dye terminator" d'Applied.

### Résultats

Le modèle exemplifié est préférentiellement le système d'oligonucléotides suivant :
Iag5 amorce sens-Iag6 amorce antisens
Iag3 sonde de révélation et Iag4 sonde de capture.
(il est à noter que Iag4 peut tout aussi bien être marquée et être utilisée en sonde de révélation).

### Etude de Spécificité

Elle a porté sur l'ensemble des souches bactériennes listées dans les Tableaux 4 et 5.

L'amplification de l'ADN extrait des 45 souches de Salmonelles testées a généré un fragment de la taille attendue (cf. Fig. 5). Les Southern blots de l'ensemble des produits amplifiés ont été hybridés avec la sonde oligonucléotidique interne Iag 3 marquée à la peroxydase. Aucune des souches non salmonelles n'a donné lieu à une hybridation avec une sonde peroxydase réalisée sur membrane selon le protocole décrit plus haut.

Les mêmes produits d'amplification ont été testés en format microplaque.

Le cut-off a été arbitrairement fixé à 0.050. Tous les représentants de chacun des groupes de Salmonelles donnent une valeur de densité optique supérieure à 0.050 (tableau 6).

### Sensibilité

Pour déterminer le nombre minimal de molécules d'ADN chromosomique de salmonelles pouvant être détecté, une gamme de dilution d'ADN chromosomique purifié a été amplifiée. 5 molécules sont visibles sur l'autoradiographie du southern blot et détectées en hybridation microplaque : la valeur obtenue en colorimétrie est supérieure au Cut-Off (Figure 6).

Les oligonucléotides sélectionnés pour la réalisation de cet exemple ont été localisés sur la séquence du gène IagA (figure 7).

**TABLEAU 4**

| SOUCHES DE SALMONELLES ETUDIEES | | | |
|---|---|---|---|
| N° | Souches | Sérotype | Groupe |
| 1 | *Salmonella Marseille* | | I |
| 2 | *Salmonella Nyanza* | | I |
| 3 | *Salmonella Poona* | | I |
| 4 | *Salmonella Kampala* | | I |
| 5 | *Salmonella Taksony* | | I |
| 6 | *Salmonella Teshie* | | I |
| 7 | *Salmonella Indiana* | | I |
| 8 | *Salmonella enteritidis* | | I |
| 9 | *Salmonella Kentucky* | | I |
| 10 | *Salmonella Napoli* | | I |
| 11 | | 841 11:a:d:en 215 | II |
| 12 | | 1703 K 41: 2: 15 | II |
| 13 | | 950 - 71 43 : d : z 39 | II |
| 14 | | 10-65 44 : 24. 223 : - | II |
| 15 | | 3209-81 45: z 23 | II |
| 16 | | 5331/ 86 62: z 29 : - | IIIa |
| 17 | | 3064-4 / 252 41: k : - | IIIa |
| 18 | | 594-54 38: z 54: - | IIIa |
| 19 | | 1694 cdai 426 63: z 4, z32: - | IIIa |
| 20 | | So 50 / 16 62: f, z 51: - | IIIa |
| 21 | | 5251-85 58: r : z 53 | IIIb |
| 22 | | 1758-76 6, 14 : z 10 : enx 215 | IIIb |
| 23 | | 453-68 16 : liv : z53 | IIIb |
| 24 | | 4305-57 16 : li(v) : z 35 | IIIb |
| 25 | | 1698-75 11 : liv : z | IIIb |
| 26 | | 8275-94 47 : r : enx 215 | IIIb |
| 27 | | 8283-94 53 : z 10 : z | IIIb |
| 28 | | cdc 456-5 / 93 40 : i : 1, 5, 7 | IIIb |
| 29 | | 8284-94 60: i : z | IIIb |
| 30 | | 1693 K 38 : k : z 55 | IIIb |
| 31 | | 1707 48 : f : z 51 : - | IV |
| 32 | | 7231 / 89 45 : z 36, z 38 | IV |
| 33 | | 6887 / 60 48 : f, z 51 : - | IV |
| 34 | | 1357 /73 43 : z4, z 24 : - | IV |
| 35 | | 1550 K 16 :z 4, z 23 : - | IV |
| 36 | *Salmonella Bongor* | 261 -66 48 : z35 : - | V |
| 37 | *Salmonella Camdeni* | 2022 - 77 44 : r : - | V |
| 38 | | 4985 - 85 48 : z 39 : - | V |
| 39 | | 7688 - 9166 : z 39 : - | V |
| 40 | | 1387 - 7340 : a : - | V |
| 41 | | 1941 - 77 6,7 : z 41: 1,7 | VI |
| 42 | | 1449 K45 : a enx | VI |
| 43 | | 4355 - 84 1.6. 14.25 : a :e,n,x | VI |
| 44 | | 1711 K 11 . b : enx | VI |
| 45 | | 1688 K 1,6.14,25 : 2 10: 1.12.7 | VI |

**TABLEAU 5**

| SOUCHES NON SALMONELLES | | |
|---|---|---|
| **N°** | **Nom** | **Identification** |
| 1 | *Klebsiella oxytoca* | 0059 SDP |
| *2* | *Klebsiella pneumoniae* | 0054 SDP |
| 3 | *Acinetobacter baumanii* | 0033 SDP |
| 4 | *Proteus mirabilis* | RP402 |
| 5 | *Serratia marcescens* | 0042 SDP |
| *6* | *Enterobacter agglomerans* | 0067 SDP |
| *7* | *Citrobacter diversus* | 0068 SDP |
| *8* | *Pseudomonas aeruginosa* | 0011 SDP |
| 9 | *Enterobacter aerogenes* | 0066 SDP |
| 10 | *Escherichia coli* | 0131 SDP |
| 11 | *Enterocoque faecalis* | 76117 |
| 12 | *Proteus mirabilis* | AP03 |
| 13 | *Enterocoque faecalis* | 76117 |
| *14* | *Enterobacter cloacae* | 0060 SDP |
| 15 | *Mycobacterium avium* | 6 |
| 16 | *Mycobacterium tuberculosis* | H 37 RV |
| 17 | *Listeria monocytogenes* | 1/2 LG3 |

**TABLEAU 6**

| DETECTION SUR MICROPLAQUE | |
|---|---|
| ECHANTILLONS | DO à 420 nm |
| 2 Nyanza gpe I | 3.029 |
| 3 Poona gpe I | 3.103 |
| 11 gpe II | 3.155 |
| 12 gpe II | 0.751 |
| 18 gpe III a | 3.139 |
| 20 gpe III a | 3.068 |
| 21 gpe III b | 3.161 |
| 30 gpe III b | 3.201 |
| 31 gpe IV | 0.272 |
| 35 gpe IV | 0.527 |
| 36 gpe V | 1.868 |
| 40 gpe V | 3.347 |
| 45 gpe VI | 0.900 |
| *Klebsiella oxytoca* | 0.022 |
| *Klebsiella pneumoniae* | 0.017 |
| *Acinetobacter baumanii* | 0.024 |
| *Proteus mirabilis* | 0.019 |
| *Serratia marcescens* | 0.019 |
| *Enterobacter agglomerans* | 0.023 |
| *Mycobacterium avium n° 6* | 0.025 |
| *Mycobacterium tuberculosis H 37 RV* | 0.020 |
| *Listeria monocytogenes*/*2 LG3* | 0.015 |
| Témoin eau | 0.018 |
| Témoin eau | 0.022 |

## Revendications

1. Acide nucléique participant à l'invasion de cellules HeLa en culture par des bactéries de l'espèce *Salmonella enterica* sous-espèce *enterica* sérovariété *Typhi* (dénommée *S. Typhi*), **caractérisé en ce qu'**il s'agit de l'acide nucléique lagA compris entre les nucléotides 97 et 1755 de la séquence représentée à la figure 1.

2. Oligonucléotide issu d'une séquence selon la revendication 1, **caractérisé en ce qu'**il consiste en l'une des séquences suivantes :

lag3: 5'-ATA TCC ACG CAG GAA ATA ACA GGA CTT-3'

lag4: 5'-GAG CGT GCC TTA CCG ACG ATA-3 '

lag5: 5'-GCA GGG ATC ACT AAG CTG TG-3'

lag6: 5'-CGT GGG CAA CCA GCA CTA ACG-3'.

3. Couple d'oligonucléotides selon la revendication 2, **caractérisé en ce qu'**il s'agit du couple suivant : lag5 (sens) : 5'-GCA GGG ATC ACT AAG CTG TG-3' et

lag6 (antisens) : 5'-CGT GGG CAA CCA GCA CTA ACG-3'.

4. Oligonucléotide capable de s'hybrider avec *Salmonella enterica* et/ou *Salmonella bongori,* **caractérisé en ce qu'**il consiste en l'un des oligonucléotides suivants et **en ce qu'**il est marqué :

lag3: 5'-ATA TCC ACG CAG GAA ATA ACA GGA CTT-3'

lag4: 5'-GAG CGT GCC TTA CCG ACG ATA-3'.

5. Oligonucléotide selon la revendication 4, **caractérisé en ce que** lag3 est une sonde de révélation de la présence d'une séquence de nucléotides amplifiée et **en ce que** lag4 est une sonde de capture d'une séquence de nucléotides amplifiée, ou vice-versa.

6. Utilisation des oligonucléotides selon la revendication 2, comme amorces pour l'amplification d'une séquence d'ADN ou d'ADNc de *Salmonella enterica* et/ou de *Salmonella bongori* comprise dans une séquence de nucléotides selon la revendication 1 ou complémentaire d'une telle séquence, ou comme sonde pour la détection d'une séquence de nucléotides amplifiée.

7. Ensemble d'oligonucléotides utilisables pour la détection de bactéries *S. enterica* et/ou *S. bongori* après amplification de l'ADN génomique ou complémentaire de *S. enterica* et/ou de *S. bongori*, **caractérisé en ce qu'**il comprend :
- un couple d'oligonucléotides selon la revendication 3, capables d'hybrider dans des conditions stringentes avec l'ADN génomique ou l'ADNc de *S. enterica* et/ou de *S. bongori*,
- une sonde selon la revendication 5.

8. Ensemble d'oligonucteotides utilisables pour la détection *in vitro* dans un échantillon biologique, de souches de Salmonella enterica et/ou de *Salmonella bongori* appartenant à l'un des groupes I, II, III, IV, V ou VI, **caractérisé en ce qu'**il contient les oligonucléotides suivants :
- la séquence lag5 (5'-GCA GGG ATC ACT AAG CTG TG-3') et lag6 (5'-CGT GGG CAA CCA GCA CTA ACG-3') utilisables en tant qu'amorces pour l'amplification et
- la séquence lag3 (5'-ATA TCC ACG CAG GAA ATA ACA GGA CTT-3') utilisable comme sonde de révélation et la séquence lag4 (5'-GAG CGT GCC TTA CCG ACG ATA-3') utilisable comme sonde de capture.

9. Protéine iagA, **caractérisée en ce qu'**elle est codée par la séquence de nucléotides lagA selon la revendication 1.

10. Protéine iagA selon la revendication 9, **caractérisée en ce qu'**elle répond à l'enchaînement d'acides aminés compris entre les acides aminés 1 et 553 de la séquence de la figure 1.

11. Procédé pour la détection *in vitro*, dans un échantillon biologique, de séquences de nucléotides *Salmonella enterica* et/ou *Salmonella bongori*, groupe I, II, II, IV, V ou VI, préalablement amplifiées, par exemple par PCR et dénaturées, **caractérisé en ce qu'**il comprend les étapes de:
- dénaturation de la séquence de *S. enterica* amplifiée,
- mise en contact des séquences de nucléotides amplifiées dénaturées, avec une sonde de capture et une sonde de révélation obtenues à partir des oligonucléotides selon l'une quelconque des revendications 2, 4 ou 5 dans des conditions permettant l'hybridation desdites sondes de capture et de révélation avec la susdite séquence de nucléotides amplifiée, la sonde de capture étant fixée à la surface d'un puits d'une plaque de microtitration et la sonde de révélation étant marquée et libre dans le tampon d'hybridation ;
- incubation du mélange réactionnel, pendant un temps suffisant pour permettre la réaction d'hybridation,
- lavage pour éliminer les oligonucléotides n'ayant pas réagi ;
- révélation des sondes de révélation ayant hybridé aux séquences de nucléotides amplifiées.

12. Procédé de détection selon la revendication 11, **caractérisé en ce que** la sonde de capture est l'oligonucléotide lag4 et la sonde de révélation est l'oligonucléotide lag3 et **en ce que** la détection est réalisée conformément aux étapes suivantes :
- dénaturation d'un volume 10 µl de la séquence amplifiée par addition volume à volume d'une solution 200 mM NaOH, 40mM EDTA,
- préhybridation des microplaques dont la surface des puits est revêtue de la sonde de capture, dans un tampon d'hybridation approprié,
- libération de la microplaque et remplissage de chacune des cupules avec 200 µl de tampon d'hybridation renfermant le fragment amplifié dénaturé et la sonde de révélation marquée à la peroxydase à la concentration de 10ng/µl,
- incubation du mélange pendant une heure à 37°C sous agitation,
- lavage du mélange ayant réagi avec une solution de lavage 10X (100mM Tris, 3M NaCl, 1 % Tween 20, pH 7,4),
- détection de l'activité de la peroxydase liée à la sonde par colorimétrie en présence d'un substrat coloré.

13. Procédé de détection selon la revendication 12, **caractérisé en ce que** l'activité de la peroxydase est détectée par mise en oeuvre des étapes suivantes :
- dépôt de 200 µl d'une solution 40 mM citrate trisodique, 0,03 % H₂O 30 %, 7,5 mg/ml d'orthophenylenediamine (OPD) dans chacun des puits contenant le mélange de réaction,
- incubation de la microplaque pendant 30 min à l'obscurité et à 37°C,
- blocage de la réaction par addition de 50 µl/puits d'une solution 4N H₂SO₄,
- détermination de la densité optique à une longueur d'onde de 492 nm (référence à 620 nm).

14. Kit pour la détection de la présence de *Salmonella enterica* et/ou de *Salmonelle bongori* dans des échantillons biologiques, comprenant :
- des oligonucléotides selon la revendication 12,
- une sonde pour la détection des fragments amplifiés, selon l'une quelconque des revendications 2, 4 ou 5, et
- les réactifs nécessaires à la réalisation de la réaction d'amplification.

15. Produit de l'amplification d'un acide nucléique de *Salmonella* par le couple d'amorces suivant :

lag5: 5'-GCA GGG ATC ACT AAG CTG TG-3'

lag6: 5'-CGT GGG CAA CCA GCA CTA ACG-3'

## Patentansprüche

1. Nukleinsäure, welche an der Invasion von HeLa-Zellen in Kultur durch Bakterien der Gattung *Salmonella enterica*, Unter-Gattung *enterica*, Serovarietät *Typhi* (genannt S. *Typhi)* beteiligt ist, **dadurch gekennzeichnet, dass** es sich um die Nukleinsäure IagA zwischen den Nukleotiden 97 und 1755 der in Figur 1 dargestellten Sequenz handelt.

2. Oligonukleotid, welches aus einer Sequenz gemäß Anspruch 1 abstammt, **dadurch gekennzeichnet, dass** es aus einer der folgenden Sequenzen besteht:

Iag3: 5'- ATA TCC ACG CAG GAA ATA ACA GGA CTT –3'

Iag4: 5'-GAG CGT GCC TTA CCG ACG ATA-3'

Iag5: 5'-GCA GGG ATC ACT AAG CTG TG-3'

Iag6: 5'-CGT GGG CAA CCA GCA CTA ACG-3'

3. Oligonukleotidpaar gemäß Anspruch 2, **dadurch gekennzeichnet, dass** es sich um das folgende Paar handelt:

Iag5 (Sense): 5'-GCA GGG ATC ACT AAG CTG TG-3'

Iag6 (Antisense): 5'-CGT GGG CAA CCA GCA CTA ACG-3'

4. Oligonukleotid, welches in der Lage ist, mit einem *Salmonella enterica* und/oder *Salmonella bongori* zu hybridisieren, **dadurch gekennzeichnet, dass** es aus einem der folgenden Oligonukleotiden besteht, und dadurch, dass es markiert ist: Iag3: 5'- ATA TCC ACG CAG GAA ATA ACA GGA CTT –3' oder

Iag4: 5'-GAG CGT GCC TTA CCG ACG ATA-3'.

5. Oligonukleotid gemäß Anspruch 4, **dadurch gekennzeichnet, dass** Iag3 eine Detektorsonde der Gegenwart einer amplifizierten Nukleotidsequenz ist, und dadurch, dass Iag4 eine Fängersonde einer amplifizierten Nukleotidsequenz ist, oder umgekehrt.

6. Verwendung von Oligonukleotiden gemäß Anspruch 2 als Starter für die Amplifikation einer DNA-Sequenz oder einer cDNA-Sequenz von *Salmonella enterica* und/oder *Salmonella bongori,* enthalten in einer Nukleotidsequenz gemäß Anspruch 1, oder Komplementär einer solchen Sequenz, oder als Sonde für die Detektion einer amplifizierten Nukleotidsequenz.

7. Satz von Oligonukleotiden, welcher verwendbar ist für die Detektion von Bakterien *S. enterica* und/oder *S. bongori* nach Amplifikation von genomischer DNA oder Komplementär-DNA von *S. enterica* und/oder *S. bongori,* **dadurch gekennzeichnet, dass** er aufweist:
- ein Oligonukleotidpaar gemäß Anspruch 3, welches in der Lage ist, unter stringenten Bedingungen mit genomischer DNA oder cDNA von *S. enterica* und/oder *S. bongori* zu hybridisieren,
- eine Sonde gemäß Anspruch 5.

8. Satz von Oligonukleotiden, welcher verwendbar ist für die in vitro-Detektion in einer biologischen Probe von Stämmen von *Salmonella enterica* und/oder *Salmonella bongori,* die zu einer der Gruppen I, II, III, IV, V oder VI gehören, **dadurch gekennzeichnet, dass** er die folgenden Oligonukleotide aufweist:
- die Sequenz Iag5 (5'-GCA GGG ATC ACT AAG CTG TG-3') und Iag6 (5'-CGT GGG CAA CCA GCA CTA ACG-3'), welche als Starter für die Amplifikation verwendbar sind, und
- die Sequenz Iag3 (5'- ATA TCC ACG CAG GAA ATA ACA GGA CTT -3'), die als Detektorsonde verwendbar ist, und die Sequenz Iag4 (5'-GAG CGT GCC TTA CCG ACG ATA-3'), die als Fängersonde verwendbar ist.

9. Protein IagA, **dadurch gekennzeichnet, dass** es durch eine Nukleotidsequenz IagA gemäß Anspruch 1 kodiert ist.

10. Protein IagA gemäß Anspruch 9, **dadurch gekennzeichnet, dass** es der Kette von Aminosäuren entspricht, die zwischen den Aminosäuren 1 und 553 der Sequenz aus Figur 1 liegen.

11. Verfahren zur *in vitro*-Detektion in einer biologischen Probe von Nukleotidsequenzen *Salmonella enterica* und/oder *Salmonella bongori*, Gruppe I, II, III, IV, V oder VI, zuvor amplifiziert, zum Beispiel durch PCR, und denaturiert, **dadurch gekennzeichnet, dass** es die folgenden Schritte aufweist:
- Denaturierung der amplifizierten Sequenz,
- in Kontakt bringen von Sequenzen denaturierter amplifizierter Nukleotide mit einer Fängersonde und einer Detektorsonde, welche von Oligonukleotiden gemäß irgendeinem der Ansprüche 2, 4 oder 5 erhalten wurden in Bedingungen, die eine Hybridisierung der Fänger- und Detektorsonde mit der amplifizierten Nukleotidsequenz ermöglichen, wobei die Fängersonde an der Oberfläche eines Napfs einer Mikrotiterplatte befestigt ist, und die Detektorsonde markiert ist und frei in dem Hybridisierungspuffer ist,
- Inkubation des Reaktionsmediums während einer Zeit, die ausreichend ist, um die Hybridisierungsreaktion zu ermöglichen,
- Waschen, um Oligonukleotide, die nicht reagiert haben, zu eliminieren,
- Detektion der Detektorsonden, die mit den amplifizierten Nukleotidsequenzen hybridisiert haben.

12. Verfahren zur Detektion gemäss Anspruch 11, **dadurch gekennzeichnet, dass** die Fängersonde das Oligonukleotid Iag4 und die Detektorsonde das Oligonukleotid Iag3 ist, und dadurch, dass die Detektion gemäss den folgenden Schritten durchgeführt wird:
- Denaturierung einer Menge von 10µl der amplifizierten Sequenz durch mengenweises Hinzufügen einer Lösung 200 mM NaOH, 40 mM EDTA,
- Prehybridisierung von Mikroplatten, deren Napfoberfläche mit der Fängersonde besetzt ist, in einem geeigneten Hybridisierungspuffer,
- Freisetzung der Mikroplatte und Füllen jedes Bechers mit 200µl des Hybridisierungspuffers, welcher das denaturierte amplifizierte Fragment enthält und die mit Peroxydase markierte Detektorsonde zu einer Konzentration von 10ng/µl,
- Inkubation der Mischung während einer Stunde bei 37°C unter Rühren,
- Waschen der Mischung, welche reagiert hat, mit einer Waschlösung 10X (100mM Tris, 3M NaCl, 1% Tween 20, pH 7,4),
- Detektion der Aktivität der Peroxydase verbunden mit der Sonde durch Kolorimetrie in Gegenwart eines kolorierten Substrats.

13. Verfahren zur Detektion gemäss Anspruch 12, **dadurch gekennzeichnet, dass** die Detektion der Aktivität der Peroxydase realisiert wird durch Durchführung der folgenden Schritte:
- Ablagerung von 200µl einer Lösung 40mM Trinatriumcitrat, 0,03% H₂0 30%, 7,5 mg/ml Orthophenylendiamin (OPD) in jeden der Näpfe, die das Reaktionsmedium enthalten,
- Inkubation der Mikroplatte während 30min bei Dunkelheit und bei 37°C,
- Blockieren der Reaktion durch Zufügen von 50µl/Napf einer Lösung 4N H₂SO₄,
- Bestimmen der optischen Dichte bei einer Wellenlänge von 492nm (Bezug auf 620nm).

14. Kit für die Detektion der Gegenwart von *Salmonella enterica* und/oder *Salmonella bongori* in biologischen Proben, welches aufweist:
- Oligonukleotide gemäß Anspruch 2,
- eine Detektorsonde für amplifizierte Fragmente gemäss irgendeinem der Ansprüche 2, 4 oder 5, und
- Reagenzien, die zur Durchführung der Amplifikationsreaktion notwendig sind.

15. Amplifikationsprodukt einer Nukleinsäure von Salmonella durch die folgenden Starterpaare:

- Iag5: 5'-GCA GGG ATC ACT AAG CTG TG-3'

- Iag6: 5'-CGT GGG CAA CCA GCA CTA ACG-3'

## Claims

1. Nucleic acid which takes part in the invasion of HeLa cells in culture by bacteria of the species *Salmonella enterica* subspecies enterica serovariety Typhi (called S. Typhi), **characterized in that** it is the nucleic acid lagA between nucleotides 97 and 1755 of the sequence represented in Figure 1.

2. Oligonucleotide derived from a sequence according to Claim 1, **characterized in that** it corresponds to one of the following sequences:

lag3: 5'-ATA TCC ACG CAG GAA ATA ACA GGA CTT-3'

lag4: 5'-GAG CGT GCC TTA CCG ACG ATA-3'

lag5: 5'-GCA GGG ATC ACT AAG CTG TG-3'

lag6: 5'-CGT GGG CAA CCA GCA CTA ACG-3'.

3. Pair of oligonucleotides according to claim 2, **characterized in that** it is the following pair: lag5 (sense): 5-GCA GGG ATC ACT AAG CTG TC-3' and

lag6 (antisense): 5'-CGT GGG CAA CCA GCA CTA ACG-3'.

4. Oligonucleotide capable of hybridizing with *Salmonella enterica* and/or *Salmonella bongori*, **characterized in that** it corresponds to one of the following oligonucleotide and **in that** it is labelled: lag3: 5-ATA TCC ACG CAG GAA ATA ACA GGA CTT-3' or

lag4: 5'-GAG CGT GCC TTA CCG ACG ATA-3'.

5. Oligonucleotide according to claim 4, **characterized in that** lag3 is a probe for revealing the presence of an amplified nucleotide sequence and **in that** lag4 is a probe for capturing an amplified nucleotide sequence, or vice-versa.

6. Use of the oligonucleotides according to claim 2, as primers for the amplification of a DNA or cDNA sequence from *Salmonella enterica* and/or from *Salmonella bongori*, which is contained in a nucleotide sequence according to Claim 1 or which is complementary to such a sequence, or as probe for the detection of an amplified nucleotide sequence.

7. Set of oligonucleotides which can be used for the detection of *S. enterica* and/or *S. bongori* bacteria after amplification of the genomic or complementary DNA of *S. enterica* and/or *S. bongori*, **characterized in that** it comprises:
- a pair of oligonucleotides according to claim 3, which are capable of hybridizing under stringent conditions with the genomic DNA or the cDNA of *S. enterica* and/or *S. bongori*,
- a probe according to claim 5.

8. Set of oligonucleotides which can be used for the *in vitro* detection, in a biological sample, of *Salmonella enterica* and/or *Salmonella Bongori* strains belonging to one of the groups I, II, III, IV, V or VI, **characterized in that** it contains the following oligonucleotides:
- the sequence lags (5'-GCA GGG ATC ACT AAG CTG TG-3') and lag6 (5'-CGT GGG CAA CCA GCA CTA ACG-3') which can be used as primers for the amplification and
- the sequence lag3 (5'-ATA TCC ACG CAG GAA ATA ACA GGA CTT-3') which can be used as a revealing probe and the sequence lag4 (5'-GAG CGT GCC TTA CCG ACG ATA-3') which can be used as a capture probe.

9. lagA protein, **characterized in that** it is encoded by the nucleotide sequence lagA according to claim 1.

10. lagA protein according to claim 9, **characterized in that** it corresponds to the amino acid chain between amino acids 1 and 553 of the sequence of Figure 1.

11. Process for the *in vitro* detection, in a biological sample, of nucleotide sequences from *Salmonella enterica* and/or *Salmonella bongori*, group I, II, III, IV, V and VI, previously amplified for example by PCR and denatured, **characterized in that** it comprises the steps of:
- denaturing the amplified sequence,
- bringing the denatured amplified nucleotide sequences into contact with a capture probe and a revealing probe which are obtained from the oligonucleotides according to any one of Claims 2, 4 or 5 under conditions allowing the hybridization of the said capture and revealing probes with the above-mentioned amplified nucleotide sequence, the capture probe being attached to the surface of a well of a microtitre plate and the revealing probe being labelled and free in the hybridization buffer;
- incubating the reaction mixture for a sufficient time to allow the hybridization reaction;
- washing in order to remove the unreacted oligonucleotides;
- revealing the revealing probes having hybridized to the amplified nucleotide sequences.

12. Detection process according to Claim 11, **characterized in that** the capture probe is the oligonucleotide lag4 and the revealing probe is the oligonucleotide lag3 and **in that** the detection is carried out in accordance with the following steps:
- denaturation of a 10 µl volume of the amplified sequence by addition, volume for volume, of a 200 mM NaOH, 40 mM EDTA solution,
- prehybridization of the microplates whose well surface is coated with the capture probe, in an appropriate hybridization buffer,
- release of the microplate and filling of each of the wells with 200 µl of hybridization buffer containing the denatured amplified fragment and the revealing probe labelled with peroxidase at the concentration of 10 ng/µl,
- incubation of the mixture for one hour at 37°C, with stirring,
- washing of the mixture which has reacted with a 10X washing solution (100 mM Tris, 3M NaCl, 1% Tween 20, pH 7.4),
- detection of the activity of the peroxidase linked to the probe by colorimetry in the presence of a colored substrate.

13. Detection process according to Claim 12, **characterized in that** the activity of the peroxidase is detected by carrying out the following steps:
- deposition of 200 µl of a 40 mM trisodium citrate solution, 0.03% H₂O 30%, 7.5 mg/ml of orthophenylenediamine (OPD) in each of the wells containing the reaction mixture,
- incubation of the microplate for 30 min in the dark and at 37°C,
- blocking of the reaction by addition of 50 µl/well of a 4N H₂SO₄ solution,
- determination of the optical density at a wavelength of 492 nm (reference at 620 nm).

14. Kit for the detection of the presence of *Salmonella enterica* and/or *Salmonella bongori*, in biological samples, comprising:
- oligonucleotides according to claim 2,
- a probe for the detection of amplified fragments, according to anyone of claims 2, 4 or 5, and
- reagents necessary for the realization of the amplification reaction.

15. Product of the amplification of a nucleic acid of *Salmonella* by the following pair of primers: lag5: 5'-GCA GGG ATC ACT AAG CTG TG-3' et
